# FASCICULE DE BREVET EUROPEEN

(11) **EP 4 572 828 B1**
(45) Date de publication et mention de la délivrance du brevet: **08.04.2026**
(21) Numéro de dépôt: 23768328.9
(22) Date de dépôt: 16.08.2023
(51) Int. Cl.: A61M 15/00

(54) **INHALATEUR DE POUDRE SÈCHE**
TROCKENPULVERINHALATOR
DRY-POWDER INHALER

(30) Priorité: 18.08.2022 FR 2208371
(43) Date de publication de la demande: 25.06.2025
(73) Titulaire: APTAR France SAS, 27110 Le Neubourg (FR)
(72) Inventeur: POULLAIN, Franck, 27400 LA HAYE MALHERBE (FR)
(74) Mandataire: CAPRI
(86) Numéro de dépôt international: PCT/FR2023/051275
(87) Numéro de publication internationale: WO 2024/038237

(56) Documents cités:
- WO-A1-02/085281
- WO-A1-03/075988
- WO-A1-2008/001132
- WO-A1-2009/121020
- WO-A1-2013/008037
- WO-A2-98/26828
- DE-A1- 102013 021 718
- US-A1- 2004 149 283

## Description

La présente invention concerne un inhalateur de poudre sèche.

Les inhalateurs de poudre sont bien connus dans l'état de la technique. Il en existe différentes sortes.

Un premier type d'inhalateur multidoses contient un réservoir recevant une multitude de doses de poudre, l'inhalateur étant pourvu de moyens de dosage permettant à chaque actionnement de séparer une dose de cette poudre du réservoir pour l'amener dans un conduit d'expulsion afin d'être distribué à l'utilisateur. Ces dispositifs nécessitent des moyens de dosage complexes et donc coûteux, et la précision et la reproductibilité du dosage ne sont pas garanties. De plus, il y a des risques de contamination de la poudre disposée dans le réservoir au fur et à mesure de l'utilisation de l'inhalateur.

Un autre type d'inhalateur multidoses consiste à prévoir plusieurs réservoirs individuels contenant chacun une dose de poudre, puis d'ouvrir un de ces réservoirs à chaque actionnement de l'inhalateur. Cette mise en œuvre assure une meilleure étanchéité de la poudre, puisque chaque dose de poudre n'est exposée à l'atmosphère qu'au moment de son expulsion. Pour réaliser ces ensembles de réservoirs individuels, diverses variantes ont déjà été proposées, telle que des bandes ou disques de blisters. Concernant l'ouverture des réservoirs individuels, il a été proposé de peler ou décoller la couche de fermeture des blisters. Ceci présente l'inconvénient d'une maîtrise difficile des forces à appliquer pour garantir une ouverture totale sans risquer d'ouvrir le réservoir suivant, particulièrement si les moyens d'ouverture doivent être actionnés par l'inhalation. Une autre solution est de percer la membrane de fermeture d'un blister à chaque actionnement, ce qui requiert des moyens de perçage complexes et donc coûteux.

Les inhalateurs multidoses décrits ci-dessus présentent de plus le problème de ne pas garantir une efficacité de la distribution optimale, avec une partie importante de la dose qui pénètre effectivement dans les poumons de l'utilisateur pour avoir un effet thérapeutique bénéfique.

Pour réaliser des dispositifs moins complexes et donc moins coûteux, il a été proposé des inhalateurs unidoses comportant un seul réservoir individuel, tels qu'un blister ou une capsule, qui est à charger dans l'inhalateur juste avant l'utilisation de celui-ci. L'avantage de ces dispositifs unidoses est qu'il n'est pas nécessaire de stocker l'ensemble des doses à l'intérieur de l'appareil, de sorte que celui-ci peut être de dimension réduite. Par contre, l'utilisation est plus compliquée pour l'utilisateur, puisque celui-ci est obligé de charger un blister ou une capsule dans l'inhalateur avant chaque utilisation. De plus, d'autres inconvénients spécifiques à ces inhalateurs unidoses existent. Ainsi, un blister ou une capsule ne permet pas la distribution de grandes doses, typiquement supérieures à 100 mg. De plus, l'ouverture, notamment des capsules ou blisters à percer, impose l'utilisation de moyens de perçage complexes, ainsi qu'un risque de résidus de membrane percée dans la poudre distribuée. Par ailleurs, les performances de vidage du réservoir ne sont pas toujours optimales, une partie de la poudre pouvant rester à l'intérieur du réservoir lors de l'inhalation.

Pour tenter de pallier à ces inconvénients, il a été proposé dans le document WO9826828 un inhalateur unidose comportant un réservoir cylindrique pourvu d'une ouverture latérale, disposé dans une chambre cylindrique de plus grand diamètre, de sorte que lors de l'inhalation, le réservoir se déplace dans la chambre selon un mouvement orbital, ce qui provoque l'expulsion de la poudre. Cette mise en œuvre permet effectivement d'améliorer l'efficacité de la distribution, avec un vidage sensiblement total du réservoir et une partie plus importante de la dose qui pénètre effectivement dans les poumons. Néanmoins, un inconvénient de ce dispositif est que la dimension du réservoir doit être bien adaptée à celle de la chambre pour assurer un bon mouvement orbital. Or, sauf à modifier l'inhalateur, ce qui serait très coûteux, ceci ne permet pas de faire varier aisément la dose de poudre, alors qu'il peut être souhaitable de pouvoir utiliser le même inhalateur avec des réservoirs contenant des dosages différents.

Les documents WO02085281A1, WO03075988A1, WO2009121020A1, WO2013008037A1 et WO2008001132A1 décrivent d'autres dispositifs de l'état de la technique.

La présente invention a pour but de fournir un inhalateur de poudre qui ne reproduit pas les inconvénients susmentionnés.

En particulier, la présente invention a pour but de fournir un tel inhalateur qui améliore l'efficacité de distribution, en permettant la distribution de la quasi-totalité de la poudre contenue dans le réservoir et en augmentant la partie de la dose qui va atteindre les poumons de l'utilisateur.

La présente invention a également pour but de fournir un tel inhalateur qui optimise le déplacement du réservoir dans la chambre lors de l'actionnement.

La présente invention a également pour but de fournir un tel inhalateur qui réduit la complexité d'utilisation pour l'utilisateur et qui garantit une meilleure sécurité d'utilisation.

La présente invention a aussi pour but de fournir un tel inhalateur qui soit simple et peu coûteux à fabriquer et à assembler.

La présente invention a donc pour objet un inhalateur de poudre sèche comportant :
- un corps et un embout buccal pourvu d'un orifice de distribution à travers lequel l'utilisateur inhale,
- une chambre,
- un réservoir contenant une dose de poudre sèche à inhaler et comportant un orifice latéral de sortie, ledit réservoir étant disposé dans ladite chambre, le diamètre dudit réservoir étant inférieur au diamètre de ladite chambre, de sorte qu'en position chargée, ledit réservoir peut se déplacer dans ladite chambre, ledit corps comportant des canaux tangentiels adaptés à amener un flux d'air d'inhalation de manière tangentielle dans ladite chambre, ce qui lors de l'inhalation génère un déplacement orbital dudit réservoir dans ladite chambre en position chargée,

ledit réservoir comportant un profil externe définissant un volume inscrit formant un volume externe du réservoir supérieur au volume interne du réservoir qui contient la poudre, ledit profil externe comportant une pluralité de nervures s'étendant radialement sur une paroi externe dudit réservoir.

Avantageusement, ledit réservoir est formé d'une partie supérieure de réservoir et d'une partie inférieure de réservoir, qui sont assemblées après remplissage avec la dose de poudre.

Avantageusement, chaque partie de réservoir comporte son profil respectif.

Avantageusement, ladite partie inférieure de réservoir comporte ledit orifice latéral de sortie.

Avantageusement, ledit embout buccal comporte une grille en amont dudit orifice de distribution.

Avantageusement, ladite chambre est disposée entre une entrée d'air d'inhalation et ledit embout buccal.

Avantageusement, ledit réservoir contient une dose de poudre supérieure à 50 mg, notamment supérieure à 100 mg.

Avantageusement, le volume occupé par ledit réservoir dans ladite chambre en position chargée est supérieur à 50% du volume de ladite chambre.

Avantageusement, le diamètre radial dudit réservoir est supérieur à sa hauteur axiale.

Avantageusement, ladite chambre est disposée dans un organe de chargement monté déplaçable, notamment pivotant, sur ledit corps entre une position non chargée, dans laquelle un réservoir peut être disposé dans ladite chambre, et une position chargée, dans laquelle ladite chambre et ledit réservoir sont disposés à l'intérieur dudit corps de l'inhalateur.

Selon une variante avantageuse, la chambre est disposée dans ledit corps, ledit inhalateur comportant un organe d'actionnement déplaçable axialement par rapport audit corps pour déplacer ledit réservoir d'une position non chargée vers une position chargée.

Avantageusement, ledit organe d'actionnement comporte au moins une rainure oblique recevant au moins un ergot respectif formé sur ledit corps ou sur un élément solidaire dudit corps, de sorte que lors de son déplacement axial, ledit organe d'actionnement réalise une rotation dans ledit corps.

Ces caractéristiques et avantages et d'autres de la présente invention apparaîtront plus clairement au cours de la description détaillée suivante, faite en référence aux dessins joints, donnés à titre d'exemples non limitatifs, sur lesquels
la figure 1 est une vue schématique en perspective d'un inhalateur selon un premier mode de réalisation avantageux, avant insertion du réservoir dans l'inhalateur,
la figure 2 est une vue similaire à celle de la figure 1, après assemblage du réservoir mais en position non chargée,
la figure 3 est une vue similaire à celle de la figure 2, en position chargée,
la figure 4 est une vue schématique en section transversale d'un inhalateur selon un second mode de réalisation avantageux, en position chargée,
la figure 5 est une vue schématique de dessous du réservoir dans la chambre, en position chargée,
la figure 6 est une vue schématique de dessus de l'embout buccal,
la figure 7 est une vue schématique en perspective du réservoir, avant fermeture,
la figure 8 est une vue schématique en section transversale du réservoir de la figure 7,
la figure 9 est une vue schématique en perspective du réservoir, après fermeture, et
la figure 10 est une vue schématique en section transversale du réservoir de la figure 9.

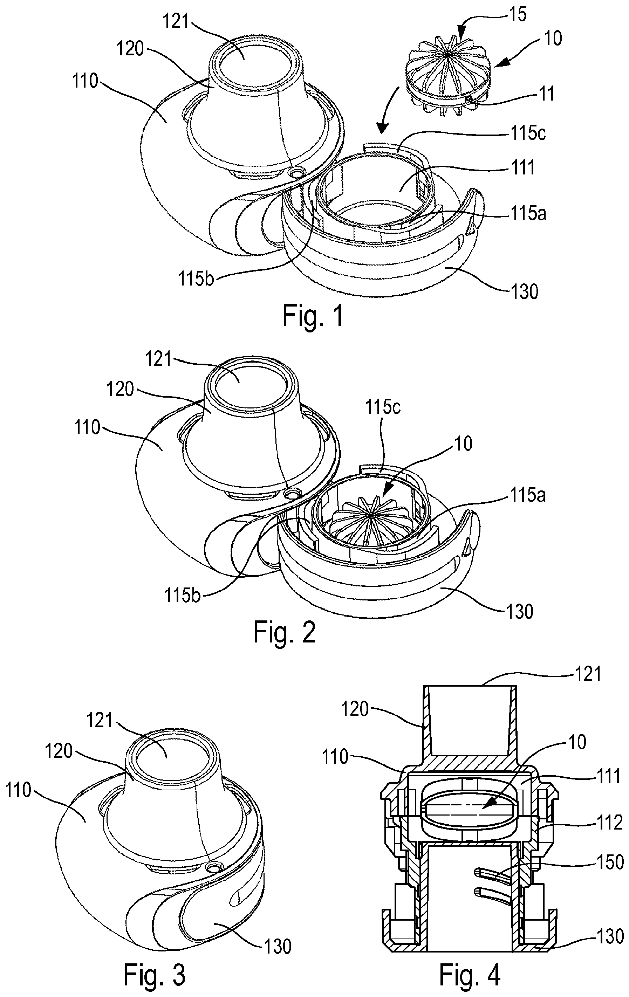

Dans la description ci-après, les termes "supérieur", "inférieur" et "latéral" se réfèrent à la position droite du dispositif représenté sur la figure 4. Les termes "axial" et "radial" se réfèrent à l'axe central longitudinal A du dispositif.

Les figures 1 à 3 décrivent un premier mode de réalisation avantageux.

Dans ce premier mode de réalisation, l'inhalateur comporte un corps 110 et un embout buccal 120 pourvu d'un orifice de distribution 121 à travers lequel l'utilisateur inhale. Un organe de chargement 130 contenant une chambre 111 est monté déplaçable, avantageusement pivotant, sur le corps 110, entre une position non chargée et une position chargée. Dans la position non chargée, un réservoir 10 peut être disposé dans la chambre 111, et dans la position chargée, représentée sur la figure 3, la chambre 111 et le réservoir 10 sont à l'intérieur du corps 110 de l'inhalateur.

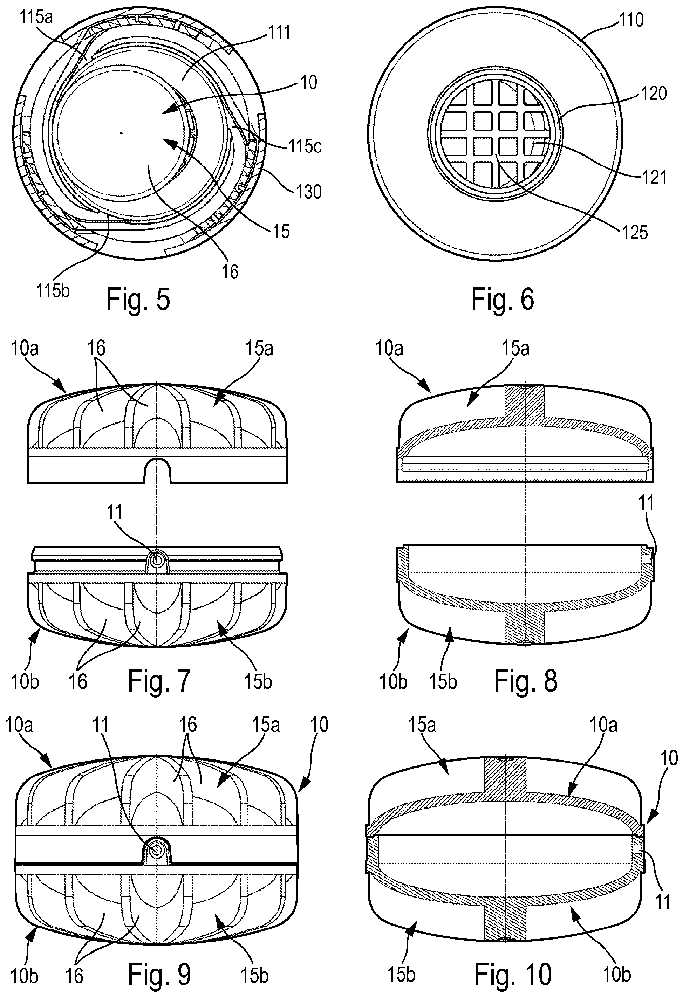

Avantageusement, comme visible sur la figure 6, l'embout buccal 120 comporte une grille 125 en amont de l'orifice de distribution 121.

De préférence, la chambre 111 est disposée entre une entrée d'air d'inhalation et ledit embout buccal 120, de sorte que lorsque l'utilisateur inhale à travers l'embout buccal 120, le flux d'air va traverser ladite chambre 111.

L'inhalateur comporte un réservoir 10 contenant une dose de poudre sèche à inhaler et comportant un orifice latéral de sortie 11.

Le diamètre du réservoir 10 est inférieur au diamètre de ladite chambre 111, de sorte qu'en position chargée, le réservoir 10 peut se déplacer dans la chambre 111. Des canaux tangentiels 115a, 115b, 115c amènent ledit flux d'air d'inhalation de manière tangentielle dans la chambre 111, ce qui va provoquer un déplacement orbital du réservoir 10 dans la chambre 111, le réservoir 10 tournant sur lui-même tout en tournant autour de la périphérie de la chambre 111 le long de la paroi latérale de celle-ci. Ce mouvement va permettre l'expulsion de la poudre contenue dans le réservoir 10, qui est entrainée par le flux d'air d'inhalation vers l'embout buccal et l'orifice de distribution.

Avantageusement, le réservoir 10 peut contenir une dose de poudre supérieure à 50 mg, notamment supérieure à 100 mg. Eventuellement, des doses très importantes peuvent être envisagées, notamment supérieures à 500 mg.

Avantageusement, le volume occupé par le réservoir 10 dans la chambre 111 en position chargée est supérieur à 50% du volume de la chambre 111.

Avantageusement, le diamètre radial du réservoir 10 est supérieur à sa hauteur axiale.

Avantageusement, le réservoir 10 est formé d'une partie supérieure de réservoir 10a et d'une partie inférieure de réservoir 10b, qui sont assemblées après remplissage avec la dose de poudre. Avantageusement, c'est la partie inférieure de réservoir 10b qui comporte l'orifice latéral de sortie 11. Avantageusement, l'orifice latéral de sortie 11 est disposé de manière axialement centrée.

Avantageusement, le réservoir 10 est symétrique, de sorte qu'il peut être inséré dans la chambre 111 indifféremment dans les deux sens.

La figure 4 décrit un second mode de réalisation avantageux.

Dans ce second mode de réalisation, l'inhalateur comporte un corps 110 contenant une chambre 111 et un embout buccal 120 pourvu d'un orifice de distribution 121 à travers lequel l'utilisateur inhale.

Avantageusement, le corps 110 est formé de deux parties de corps, une partie de corps supérieure comportant l'embout buccal 120 et une partie de corps inférieure comportant un manchon cylindrique 112, la chambre 111 étant définie par ces deux parties de corps fixées l'une à l'autre.

L'inhalateur comporte aussi un organe d'actionnement 130 pour déplacer ledit réservoir 10 de sa position non chargée vers sa position chargée. Cet organe d'actionnement 130 est déplaçable axialement par rapport au corps 110 pour pousser le réservoir 10 de sa position non chargée vers sa position chargée.

Avantageusement, le déplacement axial de l'organe d'actionnement 130 se fait par une rotation de l'organe d'actionnement 130 dans le corps 110, par exemple au moyen d'ergot(s) du corps 110 disposé(s) dans une(des) rainure(s) oblique(s) 150 de l'organe d'actionnement 130.

Selon l'invention, le réservoir 10 comporte un profil externe 15 définissant un volume externe du réservoir supérieur au volume interne du réservoir qui contient la poudre. Cette mise en œuvre permet d'optimiser la dimension externe du réservoir 10 par rapport à la dimension de la chambre 111, afin de garantir un bon mouvement orbital du réservoir 10 lors de l'actionnement. En même temps, le volume interne du réservoir peut être modifié, tout en gardant toujours le même volume externe, ce qui permet d'utiliser des réservoirs avec différents dosages de poudre dans le même inhalateur.

Avantageusement, chaque partie de réservoir 10a, 10b comporte son profil respectif 15a, 15b.

Selon l'invention, le profil externe 15 comporte une pluralité de nervures 16 s'étendant radialement sur la paroi externe du réservoir 10. Le nombre de nervures peut être quelconque, du moment qu'elles définissent un volume inscrit formant le volume externe du réservoir.

Le dispositif de l'invention est simple et efficace. Il est constitué d'un faible nombre de pièces, il est donc peu coûteux à fabriquer et à assembler, et fiable d'utilisation. Il permet une distribution optimale de la poudre de par le mouvement orbital du réservoir lors de l'actionnement, tout en garantissant l'intégrité de la poudre jusqu'à son utilisation.

Il est à noter que l'inhalateur est rechargeable, en retirant le réservoir vide pour le remplacer par un réservoir plein.

Diverses modifications sont également possibles pour un homme du métier sans sortir du cadre de la présente invention tel que défini par les revendications annexées.

## Revendications

1. Inhalateur de poudre sèche comportant :
- un corps (110) et un embout buccal (120) pourvu d'un orifice de distribution (121) à travers lequel l'utilisateur inhale,
- une chambre (111),
- un réservoir (10) contenant une dose de poudre sèche à inhaler et comportant un orifice latéral de sortie (11), ledit réservoir (10) étant disposé dans ladite chambre (111), le diamètre dudit réservoir (10) étant inférieur au diamètre de ladite chambre (111), de sorte qu'en position chargée, ledit réservoir (10) peut se déplacer dans ladite chambre (111), ledit corps (110) comportant des canaux tangentiels (115a, 115b, 115c) adaptés à amener un flux d'air d'inhalation de manière tangentielle dans ladite chambre (111), ce qui lors de l'inhalation génère un déplacement orbital dudit réservoir (10) dans ladite chambre (111) en position chargée, **caractérisé en ce que** ledit réservoir (10) comporte un profil externe (15) définissant un volume inscrit formant un volume externe du réservoir supérieur au volume interne du réservoir qui contient la poudre, ledit profil externe (15) comportant une pluralité de nervures (16) s'étendant radialement sur une paroi externe dudit réservoir (10).

2. Inhalateur selon la revendication 1, dans lequel ledit réservoir (10) est formé d'une partie supérieure de réservoir (10a) et d'une partie inférieure de réservoir (10b), qui sont assemblées après remplissage avec la dose de poudre.

3. Inhalateur selon la revendication 2, dans lequel chaque partie de réservoir (10a, 10b) comporte son profil respectif (15a, 15b).

4. Inhalateur selon la revendication 2 ou 3, dans lequel ladite partie inférieure de réservoir (10b) comporte ledit orifice latéral de sortie (11).

5. Inhalateur selon l'une quelconque des revendications précédentes, dans lequel ledit embout buccal (120) comporte une grille (125) en amont dudit orifice de distribution (121).

6. Inhalateur selon l'une quelconque des revendications précédentes, dans lequel ladite chambre (111) est disposée entre une entrée d'air d'inhalation et ledit embout buccal (120).

7. Inhalateur selon l'une quelconque des revendications précédentes, dans lequel ledit réservoir (10) contient une dose de poudre supérieure à 50 mg, notamment supérieure à 100 mg.

8. Inhalateur selon l'une quelconque des revendications précédentes, dans lequel le volume occupé par ledit réservoir (10) dans ladite chambre (111) en position chargée est supérieur à 50% du volume de ladite chambre (111).

9. Inhalateur selon l'une quelconque des revendications précédentes, dans lequel le diamètre radial dudit réservoir (10) est supérieur à sa hauteur axiale.

10. Inhalateur selon l'une quelconque des revendications précédentes, dans lequel ladite chambre (111) est disposée dans un organe de chargement (130) monté déplaçable, notamment pivotant, sur ledit corps 110 entre une position non chargée, dans laquelle un réservoir (10) peut être disposé dans ladite chambre (111), et une position chargée, dans laquelle ladite chambre (111) et ledit réservoir (10) sont disposés à l'intérieur dudit corps (110) de l'inhalateur.

11. Inhalateur selon l'une quelconque des revendications 1 à 9, dans lequel ladite chambre (111) est disposée dans ledit corps (110), ledit inhalateur comportant un organe d'actionnement (130) déplaçable axialement par rapport audit corps (110) pour déplacer ledit réservoir (10) d'une position non chargée vers une position chargée.

12. Inhalateur selon la revendication 11, dans lequel ledit organe d'actionnement (130) comporte au moins une rainure oblique (150) recevant au moins un ergot respectif formé sur ledit corps (110) ou sur un élément solidaire dudit corps (110), de sorte que lors de son déplacement axial, ledit organe d'actionnement (130) réalise une rotation dans ledit corps (110).

## Patentansprüche

1. Trockenpulverinhalator, umfassend:
- einen Körper (110) und ein Mundstück (120), das mit einer Verteileröffnung (121) versehen ist, durch die der Benutzer inhaliert,
- eine Kammer (111),
- ein Reservoir (10), das eine Dosis von zu inhalierendem Trockenpulver enthält und eine seitliche Austrittsöffnung (11) umfasst, wobei das Reservoir (10) in der Kammer (111) angeordnet ist, wobei der Durchmesser des Reservoirs (10) kleiner als der Durchmesser der Kammer (111) ist, so dass sich das Reservoir (10) in der geladenen Position in der Kammer (111) bewegen kann, wobei der Körper (110) tangentiale Kanäle (115a, 115b, 115c) umfasst, die dazu angepasst sind, einen Inhalationsluftstrom tangential in die Kammer (111) zu bringen, wodurch beim Inhalieren eine orbitale Verschiebung des Reservoirs (10) in der Kammer (111) in der geladenen Position entsteht,
**dadurch gekennzeichnet, dass** das Reservoir (10) ein externes Profil (15) umfasst, das ein eingeschriebenes Volumen definiert, das ein externes Volumen des Reservoirs bildet, das größer als das interne Volumen des Reservoirs, welches das Pulver enthält, ist, wobei das externe Profil (15) eine Vielzahl von Rippen (16) umfasst, die sich an einer externen Wand des Reservoirs (10) radial erstrecken.

2. Inhalator nach Anspruch 1, wobei das Reservoir (10) aus einem oberen Reservoirteil (10a) und einem unteren Reservoirteil (10b) gebildet ist, die nach dem Befüllen mit der Pulverdosis zusammengefügt werden.

3. Inhalator nach Anspruch 2, wobei jeder Reservoirteil (10a, 10b) sein jeweiliges Profil (15a, 15b) umfasst.

4. Inhalator nach Anspruch 2 oder 3, wobei der untere Reservoirteil (10b) die seitliche Austrittsöffnung (11) umfasst.

5. Inhalator nach einem der vorhergehenden Ansprüche, wobei das Mundstück (120) ein Gitter (125) umfasst, das der Verteileröffnung (121) vorgelagert ist.

6. Inhalator nach einem der vorhergehenden Ansprüche, wobei die Kammer (111) zwischen einem Inhalationslufteingang und dem Mundstück (120) angeordnet ist.

7. Inhalator nach einem der vorhergehenden Ansprüche, wobei das Reservoir (10) eine Pulverdosis von mehr als 50 mg, insbesondere von mehr als 100 mg enthält.

8. Inhalator nach einem der vorhergehenden Ansprüche, wobei das Volumen, das von dem Reservoir (10) in der Kammer (111) in der geladenen Position eingenommen wird, größer als 50 % des Volumens der Kammer (111) ist.

9. Inhalator nach einem der vorhergehenden Ansprüche, wobei der radiale Durchmesser des Reservoirs (10) größer als seine axiale Höhe ist.

10. Inhalator nach einem der vorhergehenden Ansprüche, wobei die Kammer (111) in einem Ladeorgan (130) angeordnet ist, das verschiebbar, insbesondere schwenkbar, an dem Körper (110) zwischen einer nicht geladenen Position, in der ein Reservoir (10) in der Kammer (111) angeordnet sein kann, und einer geladenen Position, in der die Kammer (111) und das Reservoir (10) im Innern des Körpers (110) des Inhalators angeordnet sind, montiert ist.

11. Inhalator nach einem der Ansprüche 1 bis 9, wobei die Kammer (111) in dem Körper (110) angeordnet ist, wobei der Inhalator ein Betätigungsorgan (130) umfasst, das im Verhältnis zum Körper axial verschiebbar ist, um das Reservoir (10) von einer nicht geladenen Position in eine geladene Position zu verschieben.

12. Inhalator nach Anspruch 11, wobei das Betätigungsorgan (130) mindestens eine schräge Rille (150) umfasst, die mindestens einen jeweiligen Ansatz aufnimmt, der an dem Körper (110) oder an einem Element, das mit dem Körper (110) fest verbunden ist, gebildet ist, so dass das Betätigungsorgan (130) bei seiner axialen Verschiebung eine Drehung in dem Körper (110) ausführt.

## Claims

1. A dry-powder inhaler comprising:
- a body (110) and a mouthpiece (120) provided with a dispensing orifice (121) through which the user inhales,
- a chamber (111),
- a reservoir (10) containing a dose of dry powder to be inhaled and comprising a lateral outlet opening (11), said reservoir (10) being disposed in said chamber (111), the diameter of said reservoir (10) being less than the diameter of said chamber (111), so that, in the loaded position, said reservoir (10) can move in said chamber (111), said body (110) comprising tangential channels (115a, 115b, 115c) adapted to bring a flow of inhalation air tangentially into said chamber (111), which, during inhalation, generates an orbital movement of said reservoir (10) in said chamber (111), in the loaded position,
**characterized in that** said reservoir (10) comprises an external profile 15 defining an inscribed volume forming an external volume of the reservoir that is greater than the internal volume of the reservoir that contains the powder, said external profile (15) comprising a plurality of ribs (16) extending radially on an outer wall of said reservoir (10).

2. The inhaler according to claim 1, wherein said reservoir (10) is formed by an upper reservoir portion (10a) and a lower reservoir portion (10b), which are assembled after filling with the dose of powder.

3. The inhaler according to claim 2, wherein each reservoir portion (10a, 10b) has its respective profile (15a, 15b).

4. The inhaler according to claim 2 or 3, wherein said lower reservoir portion (10b) comprises said lateral outlet opening (11).

5. The inhaler as claimed in any one of the preceding claims, wherein said mouthpiece (120) comprises a grid (125) upstream from said dispensing orifice (121).

6. The inhaler as claimed in any one of the preceding claims, wherein said chamber (111) is disposed between an inhalation air inlet and said mouthpiece (120).

7. The inhaler as claimed in any one of the preceding claims, wherein said reservoir (10) contains a dose of powder greater than 50 mg, in particular greater than 100 mg.

8. The inhaler as claimed in any one of the preceding claims, wherein the volume occupied by said reservoir (10) in said chamber (111) in the loaded position is greater than 50% of the volume of said chamber (111).

9. The inhaler as claimed in any one of the preceding claims, wherein the radial diameter of said reservoir (10) is greater than its axial height.

10. The inhaler as claimed in any one of the preceding claims,, wherein said chamber is disposed in a loading member (130) mounted so as to be movable, in particular pivoting, on said body 110 between a non-loaded position, in which a reservoir (10) can be disposed in said chamber (111), and a loaded position, in which said chamber (111) and said reservoir (10) are disposed inside said body (110) of the inhaler.

11. The inhaler as claimed in any one of claims 1 to 9, wherein said chamber (111) is disposed in said body (110), said inhaler comprising an actuating member (130) axially movable with respect to said body (110) in order to move said reservoir (10) from a non-loaded position towards a loaded position.

12. The inhaler according to claim 11, wherein said actuating member (130) comprises at least one oblique groove (150) receiving at least one respective lug formed on said body (110) or on an element which is secured to said body (110), in a manner such that during its axial displacement, said actuating member (130) implements a rotation in said body (110).
